(19) Europäisches Patentamt / European Patent Office / Office européen des brevets

(11) **EP 2 494 997 A2**

(12) **EUROPEAN PATENT APPLICATION**

(43) Date of publication:
**05.09.2012 Bulletin 2012/36**

(51) Int Cl.:
*A61M 1/02* (2006.01)

(21) Application number: **12075040.1**

(22) Date of filing: **22.09.2006**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK RS**

(30) Priority: **28.11.2005 US 287831**

(62) Document number(s) of the earlier application(s) in accordance with Art. 76 EPC:
**06825108.1 / 1 962 709**

(71) Applicant: **Hemerus Medical, LLC**
**Saint Paul, MN 55110 (US)**

(72) Inventor: **Zia, Majid**
**White Bear Township, Minnesota 55127 (US)**

(74) Representative: **Pfenning, Meinig & Partner GbR**
**Patent- und Rechtsanwälte**
**Joachimstaler Strasse 12**
**10719 Berlin (DE)**

Remarks:
This application was filed on 17-04-2012 as a divisional application to the application mentioned under INID code 62.

(54) **Prechargeable fluid filtration method and apparatus**

(57)     The invention relates, in particular, to a method for processing a biological fluid using a processing system having a first biological fluid container containing a quantity of biological fluid, a first biological fluid receiving container downstream of said first biological fluid container precharged with a quantity of gas sufficient to drain the system, and a functional biomedical device in fluid communication with the first biological fluid container and the first biological fluid receiving container, the method comprising:

a) causing the precharge of gas to be transferred from the first biological fluid receiving container to the first biological fluid container; and

b) passing the biological fluid from the first biological fluid container, through the functional biomedical device.

Fig-1

EP 2 494 997 A2

**Description**

BACKGROUND OF THE INVENTION

1. Field of the Invention

The present invention relates to a method and apparatus for processing fluids. More particularly, the present invention relates to a method and apparatus for processing biological fluids, such as donated blood, into therapeutically valuable components. Most particularly, the present invention relates to an improved method and apparatus for processing donated blood into its therapeutically valuable components which uses an improved ventless system to substantially increase the recovery of all the blood products from the donated blood or prepared blood component.

2. Discussion of the Related Art

Methods and apparatus for processing blood are well known in the prior art. U.S. Patent No. 3,892,236 to Djerassi shows an apparatus for the continuous withdrawal of blood from a human donor, forced extracorporeal circulation of blood of the donor with separation of granulocytes, and return by gravity of the leukocyte-poor whole blood to the donor.

U.S. Patent No. 5,126,054 to Matkovich shows a venting means for venting gas from the transfer line of a liquid delivery system comprising a housing, a first, liquid-wettable, microporous membrane carried in said housing so as to be in communication with the transfer line, and a second, non-liquid-wettable, gas permeable microporous membrane superimposed on said microporous membrane to the outward side of the housing. Gas in the delivery system is v,ented from the system so long as the first microporous membrane remains unwetted by the delivery liquid.

U.S. Patent No. 5,451,321 to Matkovich shows a blood processing system for the processing of blood or a blood component into valuable blood products having a first container, a second container downstream of the first container, a transfer line communicating between the first container and the second container, a leukocyte depletion filter carried in the transfer line between the first container and the second container, and having a gas outlet carried in the transfer line downstream of the leukocyte depletion filter and upstream of the second container.

U.S. Patent No. 5,472,621 to Matkovich shows a method and apparatus for treating transition zone material having a first container, a second container downstream of the first container, and a red cell barrier medium, which may be interposed between the first container and the second container. The red cell barrier medium may also be interposed between the second container and a third container. A gas collection and displacement loop is connected between the upstream side and the downstream side of the red cell barrier medium.

U.S. Patent No. 5,527,472 to Bellotti, et al. shows systems and methods for processing blood which direct blood through the inlet of a separation device for removing undesired matter while directing the blood substantially free of undesired matter from the outlet of the separation device and into a collection container through the first port.

U.S. Patent No. 5,863,436 to Matkovich shows a sterile blood processing system for the post donation processing of donated blood into valuable blood products having a first container, a second container downstream of the first container, and a leukocyte depletion filter in fluid communication with, and interposed between, the first container and the second container and having a gas inlet for allowing gas to reach the leukocyte depletion filter and displace the blood from the leukocyte depletion device, wherein the inlet is disposed between the leukocyte depletion device and the first container.

U.S. Patent No. 6,802,425 B2 to Zia, et al. shows open and closed loop biological fluid processing systems which all share the concept that the gases transferred into, or out of, or within the biological fluid processing system have the transfer lines arranged or configured in a manner which precludes the biological fluid from ever contacting the upstream and/or downstream gas inlet or outlet housings or vents, or bypassing the fluid filtration device.

While these devices are generally satisfactory, some of the methods and apparatus of the prior art leave a large amount of biological fluid trapped in various elements of the fluid processing apparatus, and they all requires gas venting devices or bypass lines of one sort or another, which makes them more complicated, and thus more expensive.

Therefore, those skilled in the art continue to search for a method and apparatus to provide for optimal recovery of the biological fluid from biological fluid processing systems, cost reduction and ease of use.

SUMMARY OF THE INVENTION

The problems of the prior art are solved by the present invention by utilizing a novel ventless and prechargeable fluid processing system. The prior art systems are vented systems because it is known for a fluid to drain out of non-collapsible components in a biological fluid processing system such as a blood bag, gas must be transferred behind a column of fluid to allow it to drain from the system. Therefore, various venting arrangements have been devised in the prior art to address this issue. However, some of the prior art devices leave a large amount of biological fluid trapped in the various elements of the fluid processing system, and they are relatively complicated and expensive to manufacture. The present invention has solved the problem in the prior art by providing a ventless system which provides for increased recovery of biological fluid by providing drainage of substantially all collapsible and non-collapsible components. By utilizing the novel idea of precharging the system with a quantity of gas, predetermined components of the system may be drained.

In one embodiment of the present invention, a fluid filtration apparatus is provided which includes a fluid filtration device having and inlet and an outlet, a first biological fluid container upstream from the fluid filtration device and having an outlet, a first conduit in fluid communication with the outlet of the first biological fluid container and the inlet of the fluid filtration device, a biological fluid receiving container downstream of the fluid filtration device and having an inlet, and a second conduit in fluid communication with the inlet of said receiving container and the outlet of said fluid filtration device. To filter a fluid using the apparatus, a precharge or excess of gas is introduced into the first biological fluid, or upstream, fluid container. The fluid in the first biological fluid container is passed through the fluid filtration device into the receiving container (by methods known in the art, such as gravitational draining) to remove undesired impurities or contaminant. Since the first biological fluid container contains a precharge or excess gas, excess gas may enter the upstream tubing and drain the fluid from desired components. It is preferred that the excess or precharge of gas be introduced or injected into the upstream fluid container shortly before the fluid is to be filtered because of the undesirability of having a gas, such as air, in contact with some types of fluid.

In another embodiment of the present invention, a fluid filtration apparatus is provided which includes a fluid filtration device having and inlet and an outlet, a fluid container upstream from the fluid filtration device and having an outlet, a first conduit in fluid communication with the outlet of the fluid container and the inlet of the fluid filtration device, a receiving container downstream of the fluid filtration device and having an inlet, a second conduit in fluid communication with the inlet of said receiving container and the outlet of said fluid filtration device, and a precharge or excess of gas sufficient to drain the system in the receiving container. To filter a fluid using the apparatus, the precharge or excess of gas is passed from the receiving container, through the fluid filtration device, without wetting out the device, and into the fluid container. The fluid in the fluid container is then passed back through the fluid filtration device into the receiving container with any desired impurities removed.

In another embodiment of the present invention, a fluid filtration apparatus is provided which includes a fluid filtration or leukocyte depletion device having an inlet and an outlet, a fluid container upstream from the fluid filtration or leukocyte depletion device and having an outlet, a first conduit in fluid communication with the outlet of said fluid container and the inlet of said fluid filtration or leukocyte depletion device, a receiving container downstream of said fluid filtration or leukocyte depletion device and having an inlet, a second conduit in fluid communication with the inlet of said receiving container and the outlet of said leukocyte depletion device, and a precharge or excess of gas sufficient to drain the system in the receiving container.

In yet another embodiment of the present invention, a biological fluid filtration apparatus is provided which includes a fluid filtration or leukocyte depletion device having an inlet and an outlet, a fluid container upstream from and elevated above said fluid filtration or leukocyte depletion device and having an outlet, a first conduit in fluid communication with the outlet of said fluid container and the inlet of said fluid filtration or leukocyte depletion device, a receiving container downstream of said fluid filtration or leukocyte depletion device and having an inlet, a second conduit in fluid communication with the inlet of said receiving container and the outlet of said leukocyte depletion device, and a precharge or excess of gas sufficient to drain the system in the receiving container. A satellite bag may be provided, which may be connected in fluid communication with the receiving container.

In still another embodiment of the invention, a method for processing a biological fluid is shown using a leukocyte reduction system having a first biological fluid container, a first biological fluid receiving container downstream of said first biological fluid container, and being precharged with a quantity of gas sufficient to drain the system, and a leukocyte reduction device in fluid communication with the first biological fluid container and the first biological fluid receiving container, the method comprising; causing the precharge of gas to be transferred from the first biological fluid receiving container to the first biological fluid container; and passing the biological fluid from the first biological fluid container, through the leukocyte reduction device, and collecting the leukocyte depleted biological fluid in the second container.

In a still further embodiment of the invention, a method for processing a leukocyte containing biological fluid is shown utilizing a leukocyte reduction system having a first biological fluid or blood container, a first biological fluid receiving container downstream of said first biological fluid container and being precharged with a quantity of gas sufficient to drain the system, and a leukocyte reduction device in fluid communication with the first biological fluid container and the first biological fluid receiving container, the method comprising: causing the precharge of gas to be transferred from the first biological fluid receiving container to the first biological fluid container; and passing the biological fluid from the first biological fluid container, through the leukocyte reduction device, and collecting the leukocyte depleted biological fluid in the first biological fluid receiving container by first inverting, and then re-inverting the apparatus.

In a further embodiment of the invention, a method for processing a biological fluid is shown including the steps of: passing a predetermined quantity of a fluid (preferably a gas such as air) from a first biological fluid receiving container through a leukocyte reduction device into a first biological fluid container containing a leukocyte rich biological fluid without wetting the device; and passing the leukocyte rich biological fluid from the first biological fluid container, through the leukocyte reduction device, into the first biological fluid receiving container by a pressure differential (e.g. gravity head, pressure cuff, suction and the like).

Thus, it is an object of the present invention to provide an improved method and apparatus for processing fluids.

A further object of the present invention is to provide a simpler and less costly apparatus for processing biological fluids.

Another object of the present invention is to provide an improved method and apparatus for filtering biological fluids.

Another object of the present invention is to provide a ventless leukocyte reduction system.

Another object of the present invention is to provide a ventless leukocyte reduction system provided with a satellite bag.

A further object of the present invention is to provide a ventless leukocyte reduction system provided with a plasma bag.

Another object of the present invention is to provide a ventless leukocyte reduction system provided with a satellite bag and one or more additive bags.

A still further object of the present invention is to provide a ventless whole blood set having a donor bag, a donor line, and a blood sampling system connected to the donor bag.

Further objects and advantages of the present invention will be apparent from the following description and appended claims, reference being had to the accompanying drawings, wherein like reference characters designate corresponding parts in the several views.

BRIEF DESCRIPTION OF THE DRAWINGS

Fig. 1 is an elevational view showing a construction embodying the present invention.

Fig.1A is a key to the various types of section lining used in Figs. 1-25.

Fig. 2 shows the construction of Fig. 1 after it has been inverted in the practice of a method in accordance with the present invention.

Fig. 3 shows the construction of Fig. 2 after the filter clamp has been opened and a precharge or excess of gas has been expressed from a first biological fluid receiving container to a first biological fluid container.

Fig. 4 shows the construction of Fig. 3 after the filter clamp has been closed and the construction has been reinverted to place the first biological fluid receiving container back in the lower position.

Fig. 5 shows the construction of Fig. 4 after the clamp has been opened and the precharge or excess of gas has driven the fluid from the first biological fluid container to the first biological fluid receiving container.

Fig. 6 is an enlarged view of the area in the view circle 6 of Fig. 5.

Fig. 7 shows the construction of Fig. 5 after the filter clamp has been closed, and the satellite bag clamp has been opened.

Fig. 8 shows the construction of Fig. 7 after any excess gas has been expressed to the satellite bag and the satellite bag clamp has been closed.

Fig. 9 is a view similar in part to Fig. 8, showing what occurs if, after the satellite bag clamp has been opened, any excess gas, together with a quantity of biological fluid, is expressed to the satellite bag before the satellite bag clamp is closed.

Fig. 10 shows the construction of Fig. 9 with the second container and the satellite bag being disconnected from the remainder of the apparatus by a sterile disconnect.

Fig. 11 shows a modification of the present invention wherein the construction of Fig. 1 is used, with the precharge or excess of gas in the first biological fluid receiving container about to be expressed to the first biological fluid container by applying pressure to the first biological fluid receiving container and opening the filter clamp 43 (and cannula 28 if not already open), in the practice of a second method in accordance with the present invention.

Fig. 12 shows the construction of Fig. 11 after the precharge or excess of gas has been expressed to the first biological fluid container, and the filter clamp has been closed.

Fig. 13 shows the construction of Fig. 12 after the filter clamp has been opened in preparation for expressing the fluid into the first biological fluid receiving container.

Fig. 14 shows the construction of Fig. 13 after the biological fluid has been transferred into the first biological fluid receiving container.

Fig. 14A is an enlarged view of the area in the view circle 14A of Fig. 14.

Fig. 15 shows the construction of Fig. 14 after the filter clamp has been closed and the satellite bag clamp has been opened, and just before pressure has been applied to the first biological fluid receiving container to drive any excess of gas into the satellite bag.

Fig. 16 shows the construction of Fig. 15 after pressure has been applied to the first biological fluid receiving container to express excess air to the satellite bag, and the satellite bag clamp has been closed.

Fig. 17 is a view similar in part to Fig. 16, showing what would happen if the excess of gas and a quantity of biological fluid was expressed before the satellite bag clamp was closed.

Fig. 18 shows the construction of Fig. 17, with the first biological fluid receiving container and the satellite bag being disconnected from the rest of the apparatus by a sterile disconnect.

Fig. 19 is an elevational view showing a modification of the present invention.

Fig. 20 is an elevational view showing a further modification of the present invention.

Fig. 20A is an elevational view showing a construction substantially the same as that illustrated in Fig. 20, but with a closable vent provided on the sampling line in place of the sterile disconnect.

Fig. 21 is an elevational view showing a still further modification of the present invention.

Fig. 21A is an elevational view showing a construction substantially the same as that illustrated in Fig. 21, but with the cannula provided at the other end of the third conduit or tubing.

Fig. 21B is an elevational view showing a construction substantially the same as that illustrated in Fig. 21, but with no cannula provided at either end of the third conduit or tubing.

Fig. 22 is an elevational view showing yet another modification of the present invention.

Fig. 22A is an elevational view showing a construction substantially the same as that illustrated in Fig. 22, but with the cannula provided at the other end of the third conduit or tubing.

Fig. 22B is an elevational view showing a construction substantially the same as that illustrated in Fig. 22, but with no cannula provided at either end of the third conduit or tubing.

Fig. 23 is an elevational view showing a still further modification of the present invention.

Fig. 23A is an elevational view showing a construction substantially the same as that illustrated in Fig. 23, but with the cannula provided at the other end of the third conduit or tubing.

Fig. 23B is an elevational view showing a construction substantially the same as that illustrated in Fig. 21, but with no cannula provided at either end of the third conduit or tubing.

Fig. 24 is an elevational view showing a modification of the present invention having a plasma bag and an additive bag.

Fig. 25 is an elevational view showing a further modification of the present invention having a plasma bag, a first additive bag, and a second additive bag.

Fig. 26 is an elevational view showing a still further modification of the present invention, wherein the precharge or excess of gas is contained in a diversion bag.

DESCRIPTION OF THE PREFERRED EMBODIMENT

In describing the present invention, the following terms are used as described below.

"Fluid" means any liquid or gas.

"Biological Fluid" refers to saline solutions, medicant solutions, nutrient solutions and blood or blood products.

"Porous Medium" refers to any porous structure through which a fluid passes. A porous medium may be formed from any synthetic or natural fiber, particulates, or from a porous or permeable membrane compatible with the fluid being filtered.

"Sterility" or "Sterile" refers to maintaining a system free from viable contaminating microorganisms.

"Connector" refers to penetrating connectors, such as a spike, cannula, or needle and mating connectors, such as Luer-type, screw-type, friction-type, or connectors which are bonded together to any structure used to form a joint or to join itself to another piece.

"Sterile Connection" refers to a joint between two tubings, or between a tubing and a device, container, or bag, which is hermetic in nature and maintains a system free from viable contaminating microorganisms.

"Sterile Disconnect" refers to any means of sealing off a "tubing" while maintaining the sterility of the contents of the tubing, if any. A heat seal is an example of a "sterile disconnect".

"Liquiphillic" refers to a material having a critical wetting surface tension higher than the surface tension of the applied liquid and is readily or spontaneously wetted by the applied liquid.

"Liquiphobic" refers to a material having a critical wetting surface tension lower than the surface tension of the applied liquid and is not readily or spontaneously wetted by the applied liquid. Liquiphobic materials may be characterized, then, by a high contact angle between a drop of liquid placed on the surface, and the surface.

"Tubing" may be any conduit or means which provides fluid communication between the containers, and is typically made from the same flexible material as is used for the containers, preferably plasticized PVC.

"Precharge" refers to a quantity of a gas introduced into the system or system component(s) prior to its use in the system.

"Ventless" refers to lacking a vent during a filtration process.

"Functional Biomedical Device" may be any number of devices or assemblies in which air or gases are present and/or may collect or form, or should be displaced prior to use of the assembly. Exemplary devices include a filter, such as a leukocyte depletion filter; a separatory device, such as a platelet concentrator, preferably a non-centrifugal platelet concentrator; a debubbler, a pump and a connector. The device may also include a device for destroying biological contaminants, such as a high intensity light wave chamber, or a device for sampling a biological liquid.

DESCRIPTION OF THE PREFERRED EMBODIMENTS

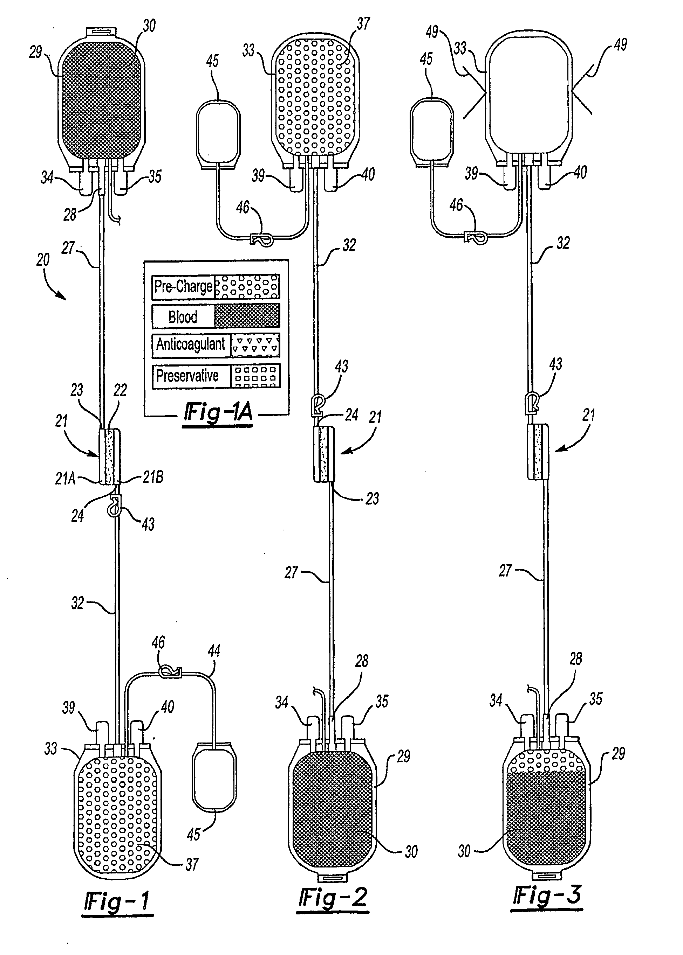

Referring now to Fig. 1, there is shown a preferred embodiment of the present invention. A first novel, prechargeable and ventless fluid processing and/or sampling apparatus or system is shown, generally designated by the numeral 20. There is shown a functional biomedical device in the form of a biological fluid filtration device 21 having an upstream chamber 21A and a downstream chamber 21 B separated by a porous medium 22 for filtering a biological fluid. Biological fluid filtration device 21 has an inlet 23 in fluid communication with the upstream chamber 21A, and an outlet 24 in fluid communication with the downstream chamber 21 B. A first tubing 27 is attached at one end to the inlet 23 of the biological fluid filtration device 21. The other end of the first tubing 27 may be connected through a cannula type first connector 28 to a first biological fluid container 29. First biological fluid container 29 may be connected to biological fluid filtration device 21 by a sterile connection, if desired. First biological fluid container 29 may be provided with a first spike port connector 34 and a second spike port connector 35.

Connected to the outlet 24 of the biological fluid filtration device 21 is a second tubing 32. A porous medium 22 is interposed between the inlet 23 and the outlet 24 of the biological fluid filtration device 21.

Connected to the other end of the second tubing 32 is a first biological fluid receiving container 33 which may have a third spike port connector 39 and a fourth spike port connector 40. Provided on the second tubing 32 proximate the outlet 24 of the biological fluid filtration device 21 may be a filter clamp 43. A hemostat may be used in place of the filter clamp 43 if desired. Hemostat or filter clamp 43 may be provided during the manufacturing process, or provided later. Filter clamp 43 is shown in an initially closed position for purposes to be described below.

Connected in fluid communication with the first biological fluid receiving container 33 by a third tubing 44 is a satellite bag 45. Provided on third tubing 44 in the manner described above may be a satellite bag clamp 46, shown in an initially closed position for purposes to be described. Satellite bag 45 is preferably empty and does not contain excess gas.

Depending on the biological fluid being filtered, the components of the ventless and prechargeable fluid processing apparatus 20 may be made of a wide variety of materials. Any practical material may be used, as long as it is compatible with the biological fluid being filtered. When blood is being filtered, it is preferable that the bag and tubing components be made of plasticized poly (vinyl chloride) (PVC) in accordance with International Standard ISO 3826: 1993 (E).

In the practice of a first method in accordance with the present invention, no further apparatus is needed. Specifically there is no precharge or excess of gas 37 in the first biological fluid receiving container 33. Instead, the excess of gas or precharge 37 is injected directly into the first biological fluid container 29. This is preferably done immediately before filtration is to take place. The excess or precharge of gas is calculated as below.

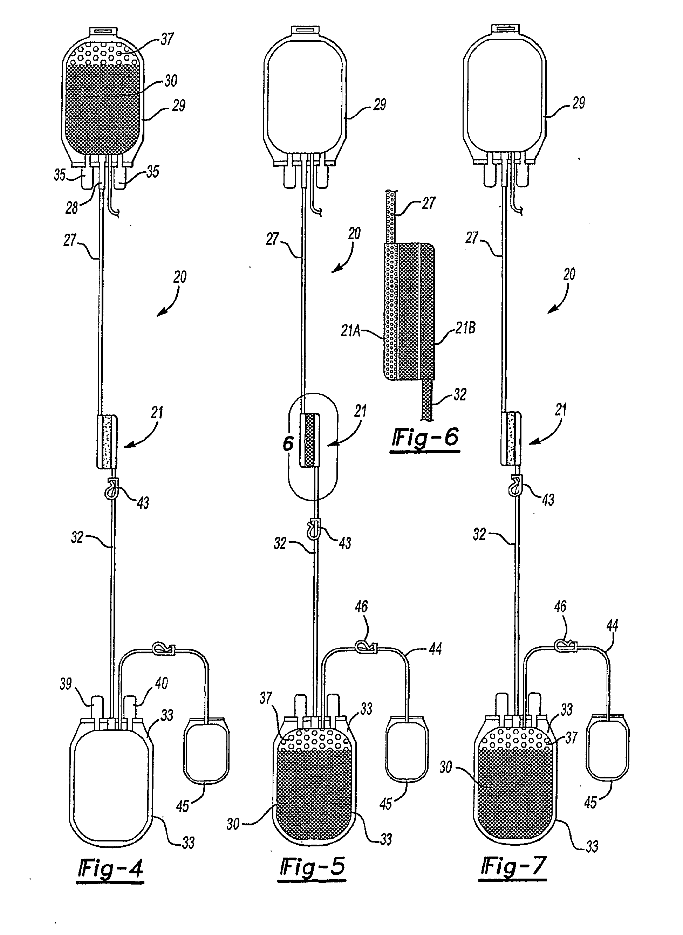

With Reference to Fig. 5, after the precharge 37 is injected or introduced into first biological fluid container 29, the filter clamp 43 is opened, (and cannula 28 if not already open) and the fluid being filtered, such as blood 30, flows through the biological fluid filtration device 21, with any desired impurities, in this case leukocytes or white blood cells removed.

In the practice of a second method embodying the present invention, a first biological fluid receiving container 33 is supplied with a precharge or excess of gas 37 in excess of the volume of gas contained in the container in its relaxed empty state (collapsed). The excess or precharge of gas 37 may be placed in the first biological fluid receiving container (33, 132) during manufacture, or injected into the first biological fluid receiving container (33, 132) before use. It is preferred that the gas being injected is sterile, and is transferred into the system in a sterile manner, such as, by passing a non-sterile gas, such as air, through a 0.2 micron filter, which is known in the art.

The volume of the precharge has to be greater than, or equal to, the volume of the residual air left during the manufacturing process in the components of the system that are intended to be drained when the first biological fluid container is emptied. The volume of gas sufficient to drain the intended portions and components of the system is hereby referred to as a volume sufficient to drain the system.

For the ventless fluid processing apparatus 20, the volume of the precharge may be obtained from the following formula:

$$V_p \geq V_{ft} + V_{uc}$$

Where,

    $V_p$ = Volume of precharge,
    $V_{ft}$ = Volume of first tubing,
    $V_{uc}$ = Volume of upstream chamber.

Therefore, the volume of the precharge or the volume of the excess gas 37 in the first biological fluid receiving container 33 is greater than or equal to the volume of the gas in the first tubing 27, plus the volume of the gas in the upstream chamber 21A. If gas is present in the first biological fluid container 29, the volume of gas in first biological fluid container 29 may be subtracted from the right hand side of the formula stated above. Therefore,

$$V_p \geq V_{ft} + V_{uc} - V_{fc}$$

Where,

    $V_{fc}$ = Volume of gas in the first biological fluid container.

Per the above explanation and formulation, no precharge 37 would be necessary if the first biological fluid container 29 contained a volume of gas greater than, or equal to the volume of tubing and portions of the functional biomedical device that it is intended to drain once the first biological fluid container 29 is drained.

This is necessary, as will be explained below, so that when the biological fluid is filtered in accordance with the method of the present invention, there will be substantially no biological fluid left in the first biological fluid container 29, the first tubing 27, or the upstream chamber 21A of the biological fluid filtration device 21. This would be the condition in the preferred embodiment of the invention.

If gas may breakthrough the biological fluid filtration device 21, it may be desired to drain additional components of the system. For example, the downstream chamber 21 B and the second tubing 32 may be drained if precharge or excess gas 37 breaks through the biological fluid filtration device 21. Typically the precharge or excess gas 37 enters the system after fluid has drained from the first biological fluid container 29, the first tubing 27, and the upstream chamber 21A. The volume of precharge may be calculated from the following formula:

$$Vp \geq Vft + Vuc + Vb + Vdc + Vst$$

Where,

Vp = Volume of precharge,
Vft = Volume of first tubing,
Vst = Volume of second tubing,
Vuc = Volume of upstream chamber,
Vdc = Volume of downstream chamber, and
Vb = Volume of biological fluid filter-volume of residual biological fluid that would remained trapped in the device.

The volume of precharge in the above example will drain additional portions of the system, such as the filter medium 22, the downstream chamber 21 B of the biological fluid filtration device 21 and the second tubing 32. This is well within the scope of the present invention. In this example, after complete drainage of the biological fluid into the first biological fluid receiving container, a substantially gas free first biological fluid receiving container may be obtained by transferring excess gas from the first biological fluid receiving container into either the satellite bag or through the biological fluid filtration device into the first biological fluid container.

Under the aforementioned International Standard ISO 3826:1993(E), the volume of air contained in the filterless blood collection pathway and the container used for the collection of blood and for each transfer container and its associated tubing shall not exceed 15 ml. The volume of air contained in each additional transfer container and associated tubing shall not exceed 15 ml. Therefore, the amount of the precharge may be calculated for each application depending on the number of containers and associated tubing, and how the system is to be drained. This requirement is intended to prevent storage of blood or blood products including additive solutions and anticoagulants with excess gas. In blood bag sets, with functional biomedical devices such as leukoreduction filters, the hold up volume of such devices is typically greater than 15 ml. Therefore, sets with functional biomedical devices typically have components which hold over 15 ml of gas (typically air) and as fluid enters the devices the air is transported out of the device typically to a container downstream of the functional biomedical device. Therefore, the excess air in the container downstream of the functional medical device must be removed.

For purposes of illustration, since the amount of residual air in the blood collection pathway and the containers is relatively small, and may become mixed with the precharge or excess gas, it is not shown separately in the following illustrations.

Referring to Figs. 1-10, there is shown the apparatus of Fig. 1 as used in the practice of another, or second, method embodying the present invention. To practice this method, and assuming the first biological fluid container 29 is filled with a desired quantity of a fluid to be filtered, which may be a biological fluid, such as blood 30, and first biological fluid receiving container 33 has precharge 37 therein, the first step is to invert the apparatus of Fig. 1, with the filter clamp 43 and the satellite bag clamp 46 in their closed position. This is shown in Fig. 2. In this position, the first biological fluid container 33 having the precharge of gas 37 is now above the first biological fluid biological fluid container 29. It is not necessary that the first biological fluid container 33 be elevated above the first biological fluid container 29, but for ease of understanding, and reducing the chances of premature liquid entry in first tubing and/or biological filtration device, the first biological fluid receiving container 33 is shown elevated vertically above the first biological fluid container 29 in this method. Also, for purposes of illustration, the biological fluid being filtered by the present invention is illustrated as blood 30, although it is well within the scope of the present invention to use any "biological fluid" as defined above.

The next step in the practice of the method, as shown in Fig. 3, is to apply a force to the outer walls of the first biological fluid receiving container 33, shown by the force arrows 49, substantially simultaneously with opening the fluid pathway between first biological fluid container 29 and first biological fluid receiving container 33 (opening filter clamp 43 and cannula 28 if not already open). This forces the precharge of gas 37 from the first biological fluid receiving container 33 through the second tubing 32, the biological fluid filtration device 21 and the first tubing 27 into the first biological fluid container 29 with the blood 30. Since first biological fluid container 29 is flexible, it expands to accommodate the volume of the precharge 37. Filter clamp 43 is then closed. An alternative method of transferring gas is to apply a vacuum around the first biological fluid container 29, and open the fluid pathway between the first biological fluid container 29 and first biological fluid receiving container 33. There are numerous methods known in the art that may be used to transfer the gas from one bag to another, and these are within the scope of the present invention.

With reference to Fig. 4, the system 20 is then re-inverted or positioned so that the first biological fluid container 29, now having the blood 30 and precharge of gas 37, is elevated above the first biological fluid receiving container 33, which is now virtually empty (does not contain any significant precharge or excess of gas).

Referring to Fig. 5, the filter clamp 43 is now opened, allowing the blood 30 from the first biological fluid container 29 to drain through the first tubing 27, the biological fluid filter 21, which for purposes of illustration is a leukocyte reduction filter, through the second tubing 32 into the first biological fluid receiving container 33. Air within these components is displaced and transferred to the first biological fluid receiving container 33.

It is preferred that the amount of excess gas or precharge 37 is chosen so that the blood 30 will drain completely from the first biological fluid container 29, first tubing 27 and the upstream chamber 21A of the biological fluid filtration device. Blood 30 will remain in the porous medium 22, the downstream chamber 21 B of the biological fluid filtration device 21 and the second tubing 32 if the differential pressure across the porous medium 22 is not sufficient to allow some gas breakthrough the porous medium. As stated previously, by choosing the volume of the precharge or excess gas 37 appropriately, it would be possible to have the blood drain completely from the filter 21 and the second tubing 32 if gas is allowed to breakthrough the porous medium but this is not believed to be the preferred embodiment. Other applications may have different amounts of excess gas 37, with different results.

With reference to Fig. 6, up to this point the contents of the first tubing 27, the second tubing 32 and the upstream and downstream chambers (21A, 21 B) of the biological fluid filtration device 21 have not been shown in detail as not being necessary to the understanding of the present invention. However, at this point in the process, it is important to understand that because of the volume of the precharge 37 which has been chosen, the upstream tubing 27 and the upstream chamber 21A of the biological fluid filtration device 21 are now filled with precharge, while the filter medium 22, the downstream chamber 21 B and the second tubing 32 are filled with blood. This condition will continue to exist in the discussion of Figs. 7-10 but again, for ease of illustration and understanding, is not shown in detail.

With reference to Fig. 7, the next step in the practice of the method is to close the filter clamp 43, and open the satellite bag clamp 46.

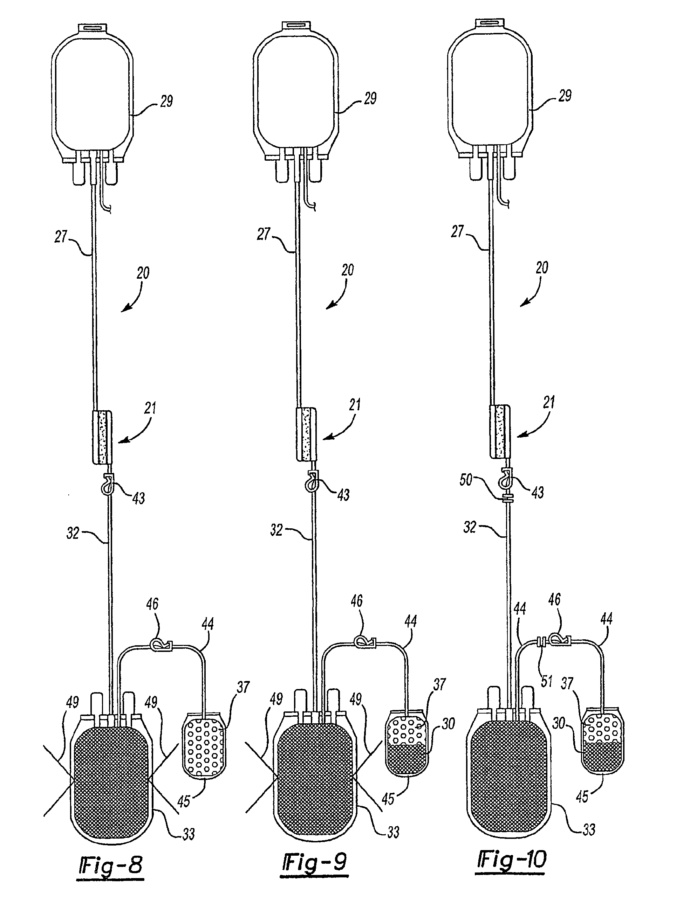

Referring now to Figs. 8 and 9, two alternatives are available. Either, as shown in Figure 8, enough force will be applied to the first biological fluid receiving container 33, as indicated by the force arrows 49, to just expel any excess gas or precharge 37 to the satellite bag 45, or, as shown in Fig. 9, enough force will be applied to the first biological fluid receiving container 33, as indicated by the force arrows 49 to expel any excess gas or precharge 37, and a desired amount of blood 30, to the satellite bag 45. The alternative shown in Fig. 9 is desirable, for example, when it is desired to test the blood 30 being filtered. In either case, the satellite bag clamp 46 is now closed.

With reference to Fig. 10, regardless of which alternative is used, the last step in the practice of the method is to make a sterile disconnect of the satellite bag 45 from the first biological fluid receiving container 33, and a sterile disconnect of the first biological fluid receiving container 33 from filtration device 21, both by means well known in the art. In the preferred embodiment, this is done by heat sealing. This is illustrated by first biological fluid sterile disconnect 50 and second sterile disconnect 51. Alternatively, after filter clamp 43 has been closed, second tubing 32 may be sterile disconnected, and excess gas transferred from first biological fluid receiving container 33 into satellite bag 45 after opening satellite bag clamp 46.

With reference to Figs. 1 and 11-18, a third method embodying the present invention is illustrated. The apparatus of Fig. 1 is used in the practice of this method. A quantity of blood 30 to be filtered is provided in first biological fluid container 29. First biological fluid container 29 is attached in fluid communication with the first tubing 27. First tubing 27 may be attached to the first biological fluid container 29 through a first cannula or connector 28. First biological fluid container 29 may be sterile connected to the first tubing 27, if desired.

It is desired that the blood 30 be filtered by biological fluid filtration device 21. An amount of excess gas or precharge 37 is provided in the first biological fluid receiving container 33. The amount of excess gas 37 is chosen as sufficient to drain the system 20 as described above. The first biological fluid receiving container 33 is connected in fluid communication with filtration device 21 by second tubing 32. There is provided a satellite bag 45, which is in fluid communication with the first biological fluid receiving container 33 through the third tubing 44. Filter clamp 43 and satellite bag clamp 46 are provided as described above.

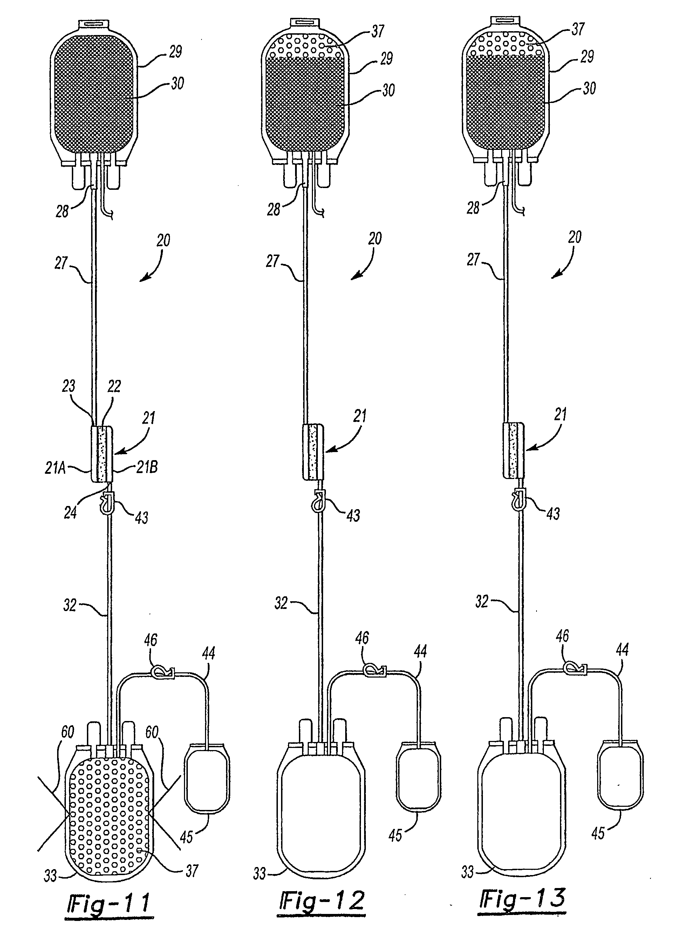

With reference to Fig. 11, the first step in the practice of the third method is to force the excess gas 37 into the first biological fluid container 29 by applying pressure to the first biological fluid container 33 as indicated by the second force arrows 60, and substantially simultaneously opening the fluid pathway between the first biological fluid container 29 and first biological fluid container 33 via the filter clamp 43 and first cannula or connector 28. The time between applying the pressure to the first biological fluid receiving container 33 and opening the filter clamp may vary somewhat. It is preferred to open the filter clamp 43 after enough pressure is applied to prevent the blood 30 from running into the first tubing 27, and possibly into the biological fluid filtration device 21, when the filter clamp 43 is opened.

This empties the first biological receiving fluid container 33 of the excess gas 37 by forcing the excess gas 37 through the second tubing 32, the biological fluid filtration device 21 and first tubing 27, into the first biological fluid container 29, without the need to invert the ventless fluid processing apparatus 20, as in the practice of the first method according to the present invention, described hereinabove.

Immediately after this operation occurs, the filter clamp 43 is closed, leaving the blood 30 and the excess gas 37 in the first biological fluid container 29. The system 20 is now in the condition shown in Fig. 12.

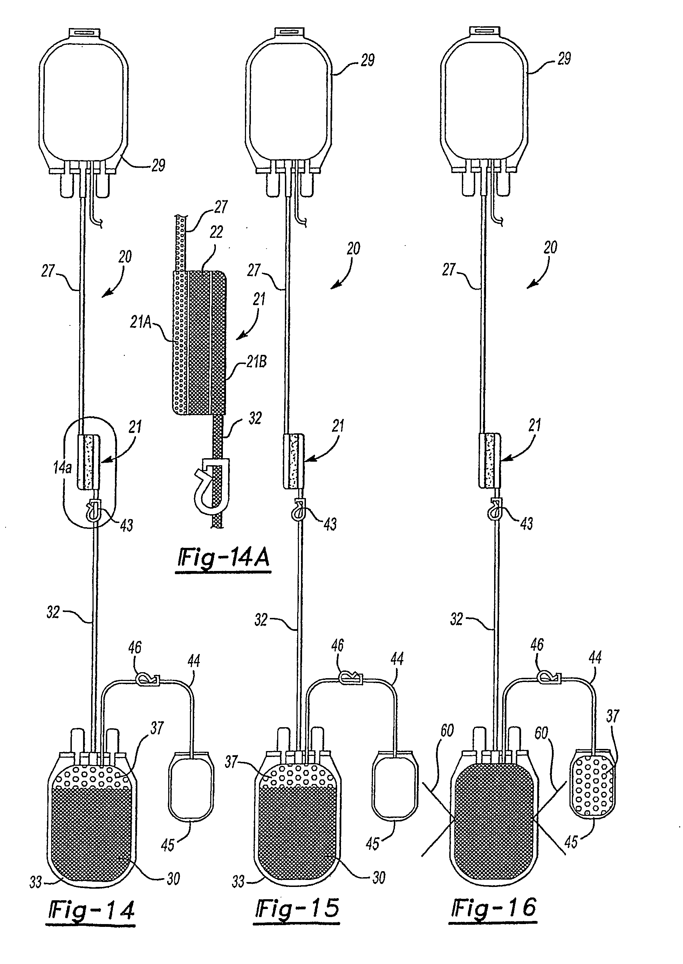

The filter clamp 43 is now opened (Fig. 13). Blood 30 will flow through the first tubing 27, the filtration device 21, the second tubing 32, and into the first biological fluid receiving container 33. Because of the excess of gas 37 being present in the first biological fluid container 29, at a minimum, the first biological fluid container 29, tubing 27 and the upstream chamber 21A of the filtration device 21 will drain and be substantially empty. Once fluid flow ceases, the filter clamp 43 is closed (Fig. 14).

With reference to Fig. 14A, up until this point, the contents of first tubing 27 and second tubing 32, as well as the upstream chamber 21A and the downstream chamber 21 B, have not been illustrated in detail as not necessary to the understanding of the invention. However, it is now important to understand that because the volume of excess gas 37 has been chosen as sufficient to drain the system, as in the method illustrated in Fig. 6, the contents of the first tubing 27 and upstream chamber 21A of the biological fluid filtration device 21 will have precharge therein, and the filter medium 22, the downstream chamber 21 B and the second tubing 32 will be filled with blood. It may be assumed that this condition exists for Figs. 15-18. It can be seen that in Figures 14 through 18 the first biological fluid container 29 is shown as empty, as if the amount of precharge was exactly equal to the amount needed to drain the system. In cases where the precharge is in excess of this amount, the first biological fluid container 29 will include a precharge of gas, which is within the scope of the present invention. As with the previous methods, if different volumes of excess gas 37 are chosen, different conditions can exist, and this is well within the scope of the present invention.

Referring to Fig. 15, the satellite bag clamp 46 is now opened to provide fluid communication between the first biological fluid receiving container 33 and the satellite bag 45.

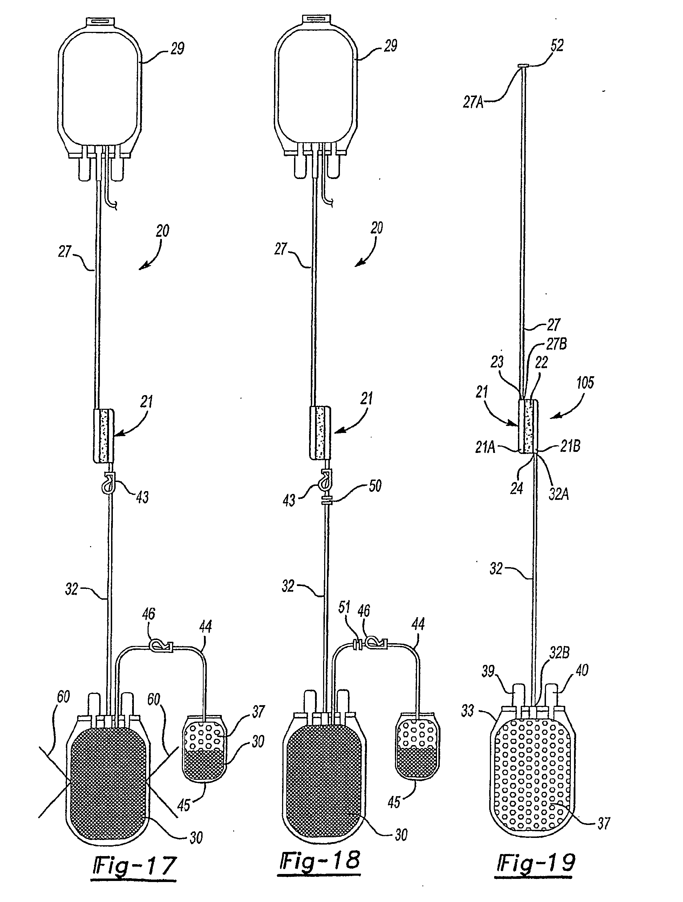

Referring to Figs. 16 and 17, as shown in connection with the previously described method, force may be applied to the first biological fluid receiving container 33 to fill the satellite bag 45 with any excess gas 37 (Fig. 16), or enough force can be applied to the first biological fluid receiving container 33 to force any excess gas 37, plus a quantity of blood 30, into the satellite bag 45 (Fig. 17). Regardless of which alternative is used, the satellite bag clamp 46 is now closed.

With reference to Fig. 18, regardless of which alternative is used, the last step in the practice of the third method is to make a sterile disconnect of the satellite bag 45 from the first biological fluid receiving container 33, and a sterile disconnect of the first biological fluid receiving container 33 from filtration device 21 by means well known in the art. In the preferred embodiment, this is done by heat sealing. A first heat seal and disconnect is made downstream of the filter clamp, as indicated by the numeral 50. A second heat seal and disconnect 51 is made upstream of the satellite bag clamp 46. Alternatively, after filter clamp 43 has been closed, second tubing 32 may be sterile disconnected, and excess gas transferred from first biological fluid receiving container 33 into satellite bag 45 after opening satellite bag clamp 46.

Now that the present invention and its principle of operation have been explained, the simple fashion and great variety of ways that apparatus for its practice may be supplied to blood banks, hospitals and other organizations concerned with the gathering and/or storage and/or administration of biological fluids may be seen.

Referring first to Fig. 19, there is shown a first apparatus 105 comprising a biological fluid filtration device 21 having an upstream chamber 21A and a downstream chamber 21 B, separated by a porous medium 22, for filtering a biological fluid. Filtration device 21 has an inlet 23 in fluid communication with the upstream chamber 21A, and an outlet 24 in fluid communication with the downstream chamber 21 B. A first tubing 27, having at least a first end 27A, and a second end 27B, is attached at its' second end 27B in fluid communication with the inlet 23 of the biological fluid filtration device 21. The first end 27A of the first tubing 27 is closed or terminated by any practical means, such as a third heat seal 52.

Connected in fluid communication with the outlet 24 of the biological fluid filtration device 21 is second tubing 32. Second tubing 32 has a first end 32A, and a second end 32B. First end 32A of tubing 32 is connected to outlet 24 by a sterile connection. The porous medium 22 is interposed between the inlet 23 and the outlet 24 of the biological fluid filtration device 21.

Connected in fluid communication with the second end 32B of the second tubing 32 by a sterile connection is first biological fluid receiving container 33 having an excess of gas 37. First biological fluid receiving container 33 may have a third spike port connector 39 and a fourth spike port connector 40.

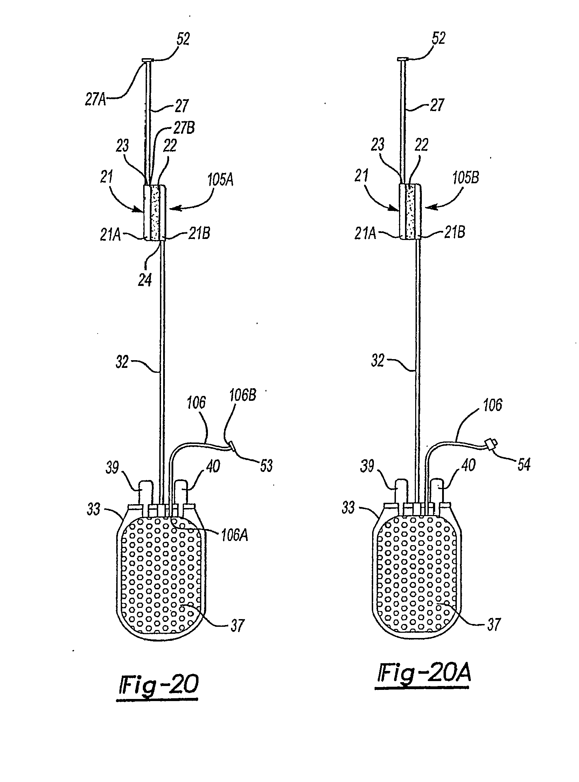

There is shown in Fig. 20 the construction shown in Fig. 19 with the addition of a sampling tubing 106, having a first end 106A and a second end 106B, to the first apparatus 105. The second apparatus 105A, therefore comprises the filtration device 21, the first tubing 27, the second tubing 32, and a first biological fluid receiving container 33, which may have a third spike port connector 39 and a fourth spike port connector 40. First tubing 27 is again terminated at its first end 27A by a third heat seal and disconnect 52, while sampling tubing 106 is terminated at its second end 106B by a fourth heat seal and disconnect 53.

Fig. 20A shows a third apparatus 105B substantially similar to second apparatus 105A shown in Fig. 20, and having the fourth heat seal and disconnect 53 replaced by a closable vent 54, which may be such as a 0.2 micron vent filter (preferably with a hydrophobic porous medium) with removable cap, or a hydrophilic and hydrophobic vent filter as described by Matkovich in patent number US 5,126,054. When the fluid receiving container 33 is equipped with a closable vent 54, it is within the scope of the present invention that the apparatus may be supplied without a precharge or excess of gas 37. In this case, the precharge or excess of gas 37 may be added, in an amount calculated as described above, to the first biological fluid receiving container 33 to drain the system upon use as described in this application.

A first biological fluid container 29 is attached to the first tubing 27, preferably by a sterile connection. Precharge or excess gas 37 is transferred from the first biological fluid receiving container 33 into the first biological fluid container 29. Subsequently, fluid is transferred from the first biological fluid container 29 through the filter 21 into the first biological fluid receiving container 33. Following the liquid transfer, precharge or excess gas 37 enters through the first tubing 27 and substantially drains the system. The initial gas volume (excess gas) transferred by the displacement of liquid from the first biological fluid container 29 into the first biological fluid receiving container 33 may be expelled from the container 33 through closeable vent 54. It is well within the skill of those in the art to determine exactly when the precharge of gas 37 should be added when using the closable vent 54.

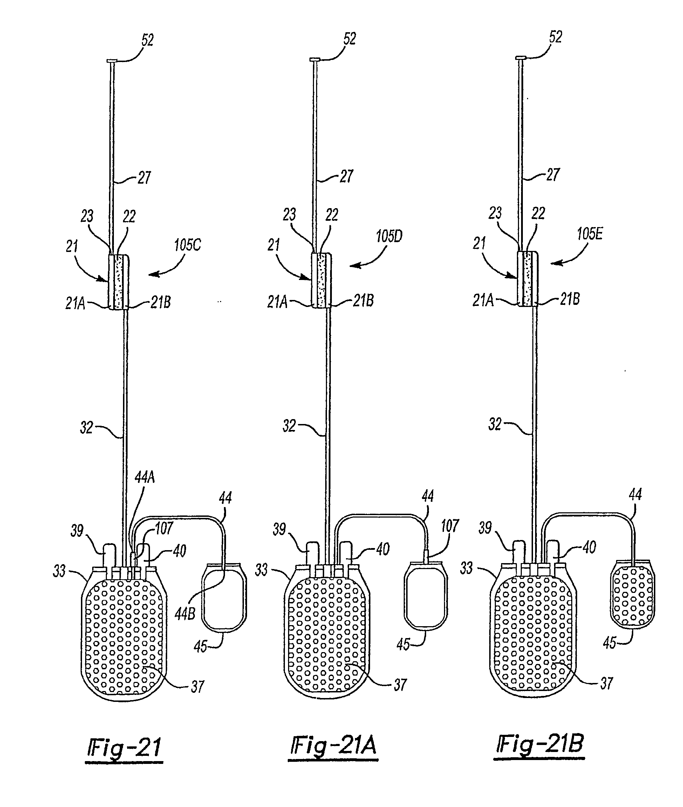

With reference to Fig. 21, there is shown fourth apparatus 105C comprising first tubing 27, biological fluid filtration device 21, second tubing 32, first biological fluid receiving container 33 having excess or precharge of gas 37, third spike port 39, and fourth spike port 40. A satellite bag 45 is provided connected to first biological fluid receiving container 33 by third or satellite bag tubing 44, having a first end 44A, and a second end 44B. First end 44A of satellite bag tubing 44 is connected in fluid communication with the first biological fluid receiving container 33 through receiving bag cannula 107. Second end 44B of satellite bag tubing 44 is connected in fluid communication with satellite bag 45.

Fig. 21A shows a fifth apparatus 105D substantially similar to fourth apparatus 105C shown in Fig. 21, and having receiving bag cannula 107 provided at the second end 44B of third tubing 44.

Fig. 21B shows a sixth apparatus 105E substantially similar to fifth apparatus 105D shown in Fig. 21A, and having no receiving bag cannula 107 provided at either end of third tubing 44. Therefore, an excess or precharge of gas 37 is also present in satellite bag 45.

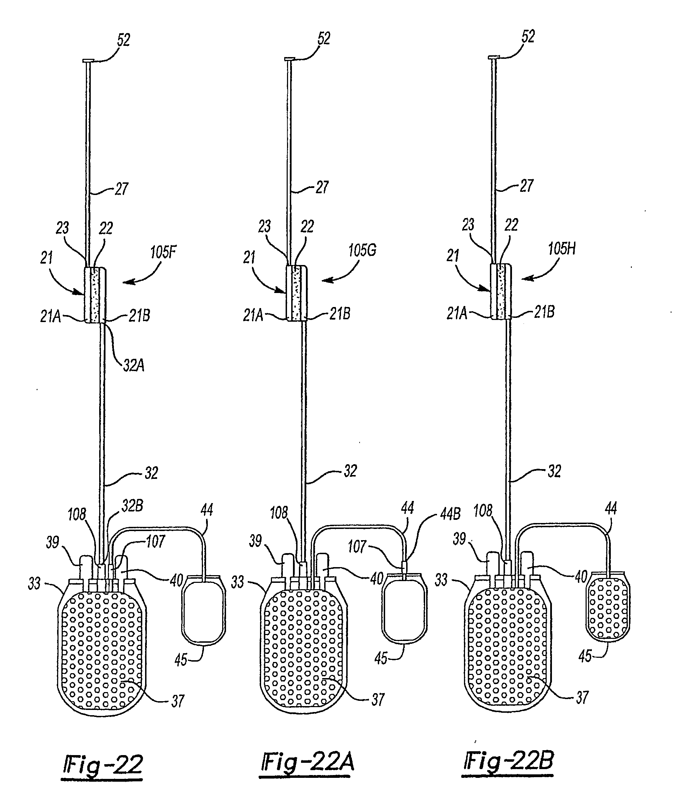

With reference to Fig. 22, the seventh apparatus 105F shown therein is substantially similar to the fourth apparatus 105C shown in Fig. 21. The only substantial difference is the addition of the second receiving bag cannula 108 interposed between the first biological fluid receiving container 33 and second end 32B of second tubing 32.

Fig. 22A shows an eighth apparatus 105G substantially similar to the seventh apparatus 105F shown in Fig. 22, and having the receiving bag cannula 107 at the second end 44B of third tubing 44, interposed between satellite bag 45 and third tubing 44.

Fig. 22B shows ninth apparatus 105H substantially similar to eighth apparatus 105G shown in Fig. 22A, and having no receiving bag cannula 107 at either end of third tubing 44. In other words, there is no cannula interposed between satellite bag 45 and third tubing 44, or between biological fluid receiving container 33 and third tubing 44.

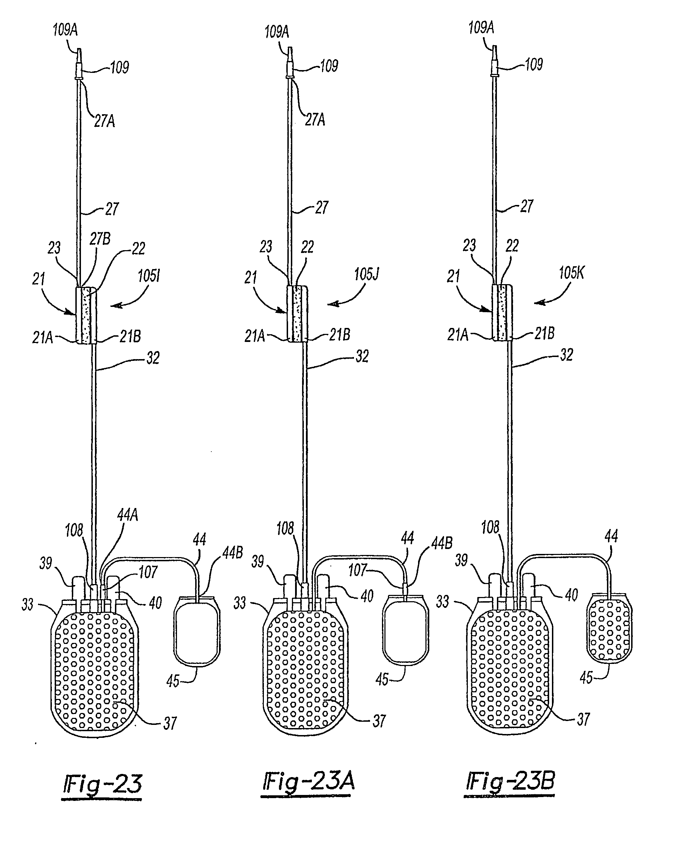

With reference to Fig. 23, tenth apparatus 105I comprises a spike 109 having spike cover 109A being connected to the upstream or first end 27A of first tubing 27. The downstream or second end 27B of first tubing 27 is connected in fluid communication with inlet 23 of biological fluid filtration device 21. The apparatus 105I further comprises a second tubing 32 connected between the downstream side of biological fluid filtration device 21 through second receiving bag cannula 108 to be in fluid communication with the interior of the biological fluid receiving container 33, which contains a precharge or excess of gas 37, which cannot be released until second receiving bag cannula 108 is opened. In tenth apparatus 105I the receiving bag cannula 107 is connected to the biological fluid receiving container 33 and, in turn is connected by third tubing 44 to satellite bag 45.

Fig. 23A shows an eleventh apparatus 105J substantially similar to tenth apparatus 105I shown in Fig. 23, and having the receiving bag cannula 107 at the second end 44B of third tubing 44, interposed between satellite bag 45 and third tubing 44.

Fig. 23B shows twelfth apparatus 105K substantially similar to eleventh apparatus 105J shown in Fig. 23A, and having no receiving bag cannula 107 at either end of third tubing 44. In other words, there is no receiving bag cannula 107 interposed between satellite bag 45 and third tubing 44, or between biological fluid receiving container 33 and third tubing 44. Therefore, an excess or precharge of gas 37 is also present in satellite bag 45.

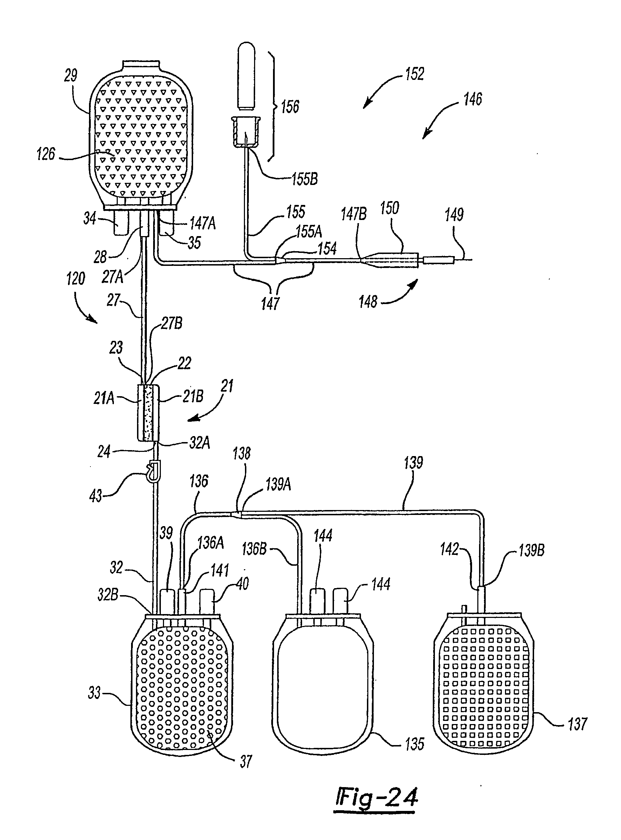

Referring to Fig. 24, there is shown a second ventless, and prechargeable, fluid processing and/or sampling apparatus, generally indicated by the numeral 120, which has proved particularly useful for the collection of biological fluids which must be later processed and/or sampled. Such apparatus may also be referred to as a "whole blood set" (WBS) 120.

The whole blood set (WBS) 120 comprises a first biological fluid container 29, which may be used to collect blood or blood product, provided upstream from biological fluid filtration device 21, which may be such as the LeukoSep™ Model No. HWB-600-W, manufactured by Hemerus Medical, LLC of St Paul, Minnesota. A quantity of anticoagulant 126 is usually provided in first biological fluid container 29.

The biological fluid filtration device 21 has an inlet 23 and an outlet 24, separated by porous medium 22. First biological fluid container is connected in fluid communication with the biological fluid filtration device 21 through first cannula or connector 28 and first tubing 27 having a first end 27A and a second end 27B.

There is also provided a first biological fluid receiving container 33 downstream of the biological fluid filtration device 21, which may be used to receive blood or blood products after they have been filtered or leukocyte reduced by the biological fluid filtration device 21. Biological fluid filtration device 21 is connected in fluid communication with first biological fluid receiving container 33 through second tubing 32, having a first end 32A and a second end 32B.

If the fluid or fluids, such as blood or blood products, in the first biological fluid receiving container 33 are to be separated into component parts, a plasma bag 135 may be provided in fluid communication with first biological fluid receiving container 33 through red blood cell (RBC) cannula 141 (when opened). Plasma bag 135 is connected to the first biological fluid receiving container 33 by red blood cell (RBC) tubing 136 (and Y-connector 138 and plasma line 136B, if needed).

In some applications, one or more additives may be added to the fluid in the first biological fluid receiving container 33 during processing. This may be accomplished by providing a first additive bag 137. First additive bag 137 is connected in fluid communication with RBC tubing 136 by primary additive tubing 139 and Y-connector 138 (when first additive bag cannula 142 is opened).

It may be desirable for one or more of the containers described above (29, 33, 135, 137) to have a cannula provided between the bag or container and its' associated conduit or tubing. For example, first biological fluid container 29 may have a first cannula or connector 28 interposed between first biological fluid container 29 and the first end 27A of first tubing 27. Blood could not pass from first biological fluid container 29 to first tubing 27 (or vice versa) until first cannula 28 was broken or otherwise compromised. It is well within the scope of the present invention to have first cannula 28 replaced by any other practical device for controlling blood flow.

Likewise, an RBC cannula 141 may be interposed between first biological fluid receiving container 33 and first end 136A of RBC tubing 136. A first additive bag cannula 142 may be interposed between first additive bag 137 and second end 139B of primary additive tubing 139. Additional cannulas or connectors may be added, depending on the particular application, and this is within the scope of the present invention. In all cases, the system may be assembled by connecting (with sterile connections, if desired) various containers and sets together.

It may be desirable to have one or more whole blood set spike ports or connectors provided on the containers (135 and/or 137) described above.

The biological fluid processing system or whole blood set 120 described above preferably includes donor apparatus 146 in connection therewith. Such an apparatus would include a donor line 147 in fluid communication with the first biological fluid container 29 at its first end 147A, and with a needle set 148 at its second end 147B. Needle set 148 includes a needle 149 in fluid communication with the donor line 147, and a needle protector 150. Donor apparatus 146 is illustrated by way of example only. It is well within the scope of the present invention to use other donor apparatus or sets known in the art.

A blood sampling system 152 may also be provided to, for example, provide a donor blood sample for testing, or to divert an initial blood sample before it is allowed to pass into first biological fluid container 29. Sampling system 152 may include such as a sampling Y-connector 154 interposed in donor line 147. Sampling tubing 155 would be connected at a first end 155A to Y-connector 154, and at its second end 155B to a vacuum tube holder 156 or other desired way to collect blood for testing, such as a pouch, or combinations of the two. Other sampling systems known in the art may also be used.

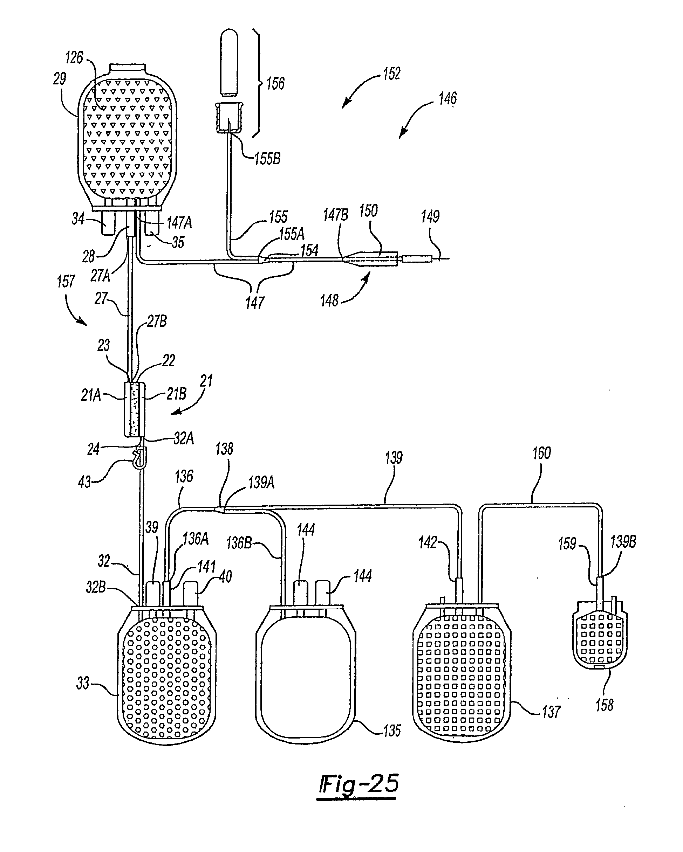

Referring now to Fig. 25, there is shown a third ventless, and prechargeable, fluid processing and/or sampling apparatus, which may also be referred to as second whole blood set 157, which is nearly identical to second ventless, and prechargeable fluid processing and/or sampling apparatus or whole blood set 120. The only substantial difference between the two is the addition of a second additive bag 158 for those applications where the processing of a biological fluid requires two or more additives. It is well within the scope of the present invention, given the teachings of the present invention, to add as many additive bags as are necessary.

Second additive bag 158 is provided, and is in fluid communication with first additive bag 137 through second additive bag cannula or connector 159 (when open) and secondary additive tubing 160.

All three methods described above may be practiced with the apparatus illustrated in Figs. 24 and 25. In the practice of the first method, a precharge or excess of gas 37, the amount of which was calculated as described above, would be injected or introduced into first biological fluid container 29 by means well known in the art before or after it is filled with blood by the donor. This would permit the blood and anti-coagulant 126 to flow through first tubing 27, and through biological fluid filtration device 21, into first biological fluid receiving container 33.

If first biological fluid receiving container 33 is provided with an excess of gas or precharge 37 as described above, blood has been introduced into first biological fluid container 29, donor cannula 129 is operated as described for cannula 28, filter clamp 43 is operated as previously described, and RBC bag cannula 141 is operated as described for the satellite clamp 46 above, the second method embodying the present invention may be practiced in connection with the apparatus as shown and described in Figs. 24 and 25. The first biological fluid receiving container 33, the plasma bag 135, the first additive bag 137, and the second additive bag 158 (if used) and subsequent additive bags, would be placed or held one next to each other, by means well known in the art, while the biological fluid processing system 157 was inverted or raised in the practice of the second method described above, with all other steps remaining the same. No change in the position of the components shown is necessary to practice the third method described above.

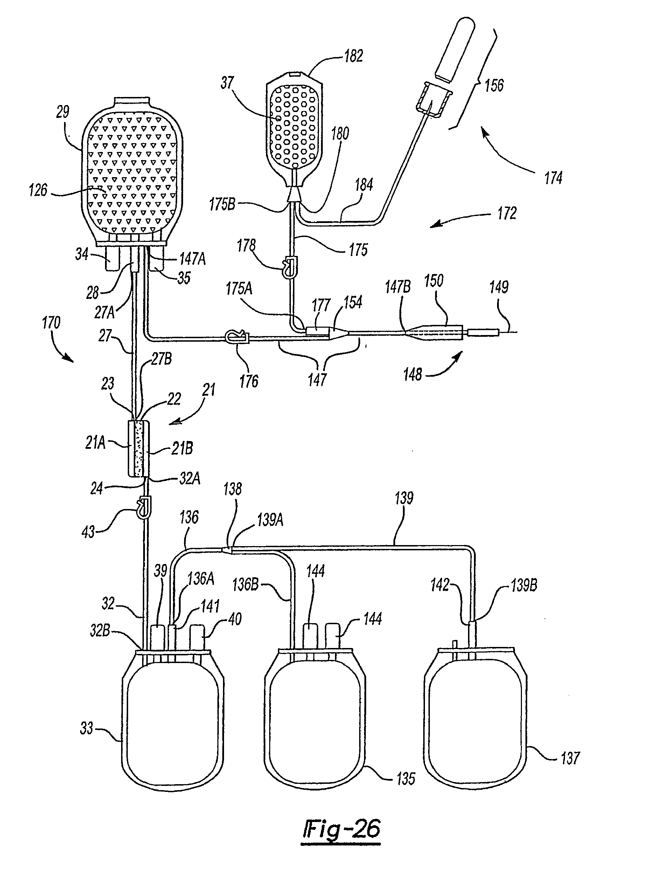

Referring now to Fig. 26, there is shown a fourth ventless, and prechargeable fluid processing and/or sampling apparatus 170. The use of fourth ventless, and prechargeable fluid processing and/or sampling apparatus 170 enables the practice of a fourth method embodying the present invention. The apparatus of Fig. 26 is similar in large part to the apparatus of Fig. 24 except for the donor apparatus 146 and the blood sampling system 152.

In this modification of the invention, second donor apparatus 172 is used in connection therewith. Such an apparatus would include, as before, a donor line 147 in fluid communication with the first biological fluid container 29 at its first end 147A, and with a needle set 148 at its second end 147B. Needle set 148 includes a needle 149 in fluid communication with the donor line 147, and a needle protector 150 to cover the needle 150 after use. A donor line clamp 176 is provided on the donor line between the needle 149 and the first biological fluid container 29. Second donor apparatus 172 is illustrated by way of example only. It is well within the scope of the present invention to use other donor apparatus or sets known in the art.

A second blood sampling system 174 is provided. Second sampling system 174 may include such as a sampling Y-connector 154 interposed in donor line 147. However, in this modification of the invention, instead of the sampling tubing 155 being connected at a first end 155A to Y-Connector 154, and at its second end 155B to a vacuum tube holder 156 or other desired way to collect blood for testing, a second sampling tubing 175 is connected at its' first end 175A to sampling cannula 177 which, in turn, is connected to the Y-connector 154.

Second sampling tubing 175 is connected at its second end 175B to a second Y-connector 180. Second Y-connector 180 is connected in a manner known in the art to provide fluid communication between diversion bag 182, the second end 175B of second sampling tubing 175, and one end of third sampling tubing 184. Third sampling tubing is connected at its other end to vacuum tube holder 156. A sampling system clamp 178 is provided on the second sampling tubing 175 between the Y-Connector 154 and the second Y-Connector 180.

While anti-coagulant 126 may be contained in first biological fluid container 29, if desired, in this modification of the invention, no precharge 37 is contained in first biological fluid receiving container 33. Instead, the precharge 37, in an amount calculated as set forth above, in other words, in an amount sufficient to drain the system, is contained in diversion bag 182.

With the above description of the fourth whole blood set 170, the fourth method according to the present invention may be understood by those skilled in the art. With donor line clamp 176 and second sampling system clamp 178 initially open, the precharge 37 in the diversion bag 182 is expressed into the first biological fluid container 29, after sampling cannula 177 is compromised. This is preferably done before, but may be done after, blood is collected in first biological fluid container 29. Because of the design of the donor needle 149 and the vacuum tube holder 156, none of the precharge can pass out of these devices until they are prepared for use. Therefore, the precharge travels through the second sampling tubing 175, sampling cannula 177, sampling Y-connector 154, and through the donor line 147, into the first biological fluid container 29. The donor line clamp 176 is then closed.

The donor needle 149 is inserted into the donor and the sampling system clamp 178 is left open. An initial amount of blood is drawn into the diversion bag 182 (and/or into the vacuum tube that may be connected to vacuum tube holder 156 if desired) for purposes known in the art, and the sampling system clamp 178 is closed. In this condition, no more blood is flowing from the donor into the fourth whole blood set 170.

Donation is continued by opening the donor line clamp 176. Blood then resumes flow into the first biological fluid container 29, where it displaces the precharge, and mixes with the anti-coagulant, if any. Once sufficient blood is collected, donor line clamp 176 is closed, and the needle 149 is removed from the donor and pulled into needle protector 150 to prevent accidental needle stick. The donor line 176 may then be sterile disconnected (and sterile segmented, if desired) from first biological fluid container 29.

To process the blood, the filter clamp 43 is then opened, permitting the blood to flow through the biological fluid filtration device 21, with the rest of the processing taking place as described above.

Therefore, by carefully studying the problems present in previous biological fluid filtration systems, I have developed a novel, method and apparatus for processing biological fluid.

In accordance with the provisions of the patent statutes, the present invention has been described in what is considered to be its preferred embodiment. However, it should be noted that the invention can be practiced otherwise than as specifically illustrated and described without departing from its spirit or scope.

The invention relates, inter alia, to the following aspects:

1. A method for processing a biological fluid using a processing system having a first biological fluid container containing a quantity of biological fluid, a first biological fluid receiving container downstream of said first biological fluid container precharged with a quantity of gas sufficient to drain the system, and a functional biomedical device in fluid communication with the first biological fluid container and the first biological fluid receiving container, the method comprising:

a) causing the precharge of gas to be transferred from the first biological fluid receiving container to the first biological fluid container; and
b) passing the biological fluid from the first biological fluid container, through the functional biomedical device.

2. A method for processing a leukocyte containing biological fluid using a leukocyte reduction system having a first biological fluid container containing a quantity of a leukocyte containing biological fluid, a first biological fluid receiving container downstream of said first biological fluid container precharged with a quantity of gas sufficient to drain the system, and a leukocyte reduction device in fluid communication with the first biological fluid container and the first biological fluid receiving container, the method comprising:

a) causing the precharge of gas to be transferred from the first biological fluid receiving container to the first biological fluid container; and
b) passing the leukocyte containing biological fluid from the first biological fluid container, through the leukocyte reduction device.

3. A method for processing a biological fluid comprising:

a) passing a predetermined quantity of gas from a first biological fluid receiving container through a leukocyte reduction device into a first biological fluid container containing a leukocyte rich biological fluid; and
b) passing the leukocyte rich biological fluid from the first biological fluid container, through the leukocyte reduction device, into the first biological fluid receiving container.

4. A prechargeable fluid filtration apparatus comprising:

a) a functional biomedical device having an inlet and an outlet and a porous medium interposed between the inlet and the outlet;
b) a first tubing connected at one end in fluid communication with the inlet of the functional biomedical device;
c) a second tubing connected at one end in fluid communication with the outlet of the functional biomedical device;
d) a first biological fluid receiving container connected in fluid communication with the other end of the second tubing; and
e) a precharge of gas in the first biological fluid receiving container sufficient to drain the system.

5. The system defined in aspect4 further comprising:

a) at least one sampling tubing having at least a first end and a second end and being connected at the first end in fluid communication with the precharge of gas in the first biological fluid container, and closed at the second end.

6. The system defined in aspect 4 further comprising:

a) at least one sampling tubing having at least a first end and a second end and connected at the first end in fluid communication with the precharge of gas in the first biological fluid container, and closed at the second end by a closable vent.

7. The system defined in aspect 4 further comprising:

a) at least one satellite bag; and
b) at least one third tubing having at least a first end and a second end and connected at the first end in fluid communication with the interior of the at least one first biological fluid receiving container, and at the second end with the interior of the at least one satellite bag.

8. The system defined in aspect 7, further comprising:

a) a receiving bag cannula interposed between the first end of the at least one third tubing and the first biological fluid receiving container.

9. The system defined in aspect 7, further comprising:

a) a receiving bag cannula interposed between the second end of the at least one third tubing and the at least one satellite bag..

10. The system defined in aspect 7 further comprising:

a) a receiving bag cannula interposed between the second end of the at least one second tubing and the first biological fluid receiving container.

11. The system defined in aspect 8 further comprising:

a) a second receiving bag cannula interposed between the second end of the at least one second tubing and the first biological fluid receiving container.

12. The system defined in aspect 9 further comprising:

a) a second receiving bag cannula interposed between the second end of the at least one second tubing and the first biological fluid receiving container.

13. The system defined in aspect 10 further comprising:

a) a spike connected at the first end of the at least one first tubing.

14. The system defined in aspect 11 further comprising:

a) a spike connected at the first end of the at least one first tubing.

15. The system defined in aspect 12 further comprising:

a) a spike connected at the first end of the at least one first tubing.

16. The system defined in aspect 4, further comprising:

a) the precharge of gas is greater than, or equal to, the residual air found in the ventless biological fluid processing system in an empty collapsed state.

17. The system defined in aspect4, further comprising:

a) a plasma bag connected to the first biological fluid receiving container.

18. The system defined in aspect 17, further comprising:

a) a first additive bag connected to the at first biological fluid receiving container.

19. The system defined in aspect 18, further comprising:

a) a second additive bag connected to the first additive bag.

20. The system defined in aspect 19 further comprising:

a) a first biological fluid container connected in fluid communication with the other end of the first tubing; and
b) a donor line having at least a first end and a second end, and connected at its' first end to the first biological fluid container.

21. The system defined in aspect20 further comprising:

a) a sampling system connected to the donor line between the first end and the second end of the donor line.

22. The system defined in aspect 20 further comprising:

a) a second sampling system connected to the donor line between the first end and the second end of the donor line.

23. The system defined in aspect 22, wherein the second sampling system comprises:

a) a sampling Y- connector interposed in the donor line between the first end and the second end of the donor line;
b) a sampling cannula connected to one branch of the sampling Y-connector
c) a diversion bag in fluid communication with the donor line when the sampling cannula is compromised; and
d) a blood gathering device in fluid communication with the donor line when the sampling cannula is compromised.

24. The system defined in aspect 21 further comprising:

a) an anti-coagulant in the first biological fluid container.

25. The system defined in aspect 23 further comprising:

a) a precharge in the diversion bag instead of or in addition to first biological fluid receiving container.

26. A ventless biological fluid processing system comprising:

a) a functional biomedical device having an inlet and an outlet and a porous medium interposed between said inlet and said outlet;
b) a first tubing having at least a first end and a second end, the second end of the first tubing connected to the inlet of the functional biomedical;
c) a second tubing having at least a first end and a second end, and being connected at the first end in fluid communication with the outlet of the functional biomedical device;
d) a first biological fluid container connected in fluid communication with the first end of the first tubing;
e) a first biological fluid receiving container connected in fluid communication with the second end of the second tubing; and
f) a precharge of gas in the first biological fluid receiving container.

27. The system defined in aspect 26 further comprising:

a) blood in the first biological fluid container.

28. The system defined in aspect 27, further comprising:

a) anti-coagulant in the first biological fluid container.

29. The system defined in aspect 28, further comprising:

a) a filter clamp on the second tubing downstream of the functional biomedical device.

30. The system defined in aspect 26, further comprising:

a) a third tubing having a first end and a second end and connected at the first end in fluid communication with the first biological fluid receiving container; and
b) a satellite bag connected in fluid communication with the second end of the third tubing.

31. The system defined in aspect 30, further comprising:

a) a satellite bag clamp on the third tubing between the first end and the second end.

32. The system defined in aspect 31, further comprising:

a) a filter clamp on the second tubing downstream of the functional biomedical device.

33. A method for processing a biological fluid using a processing system having a first biological fluid container containing a quantity of biological fluid, a first biological fluid receiving container downstream of said first biological fluid container precharged with a quantity of gas sufficient to drain the system, and a functional biomedical device in fluid communication with the first biological fluid container and the first biological fluid receiving container, the method comprising:

a) causing the precharge of gas to be transferred from the first biological fluid receiving container to the first biological fluid container by applying a vacuum about the first biological fluid container; and
b) passing the biological fluid from the first biological fluid container, through the functional biomedical device, and collecting the biological fluid in the first biological fluid receiving container.

34. A method for processing a fluid using a processing system having a first biological fluid container containing a quantity of biological fluid, a first biological fluid receiving container downstream of said first biological fluid container precharged with a quantity of gas sufficient to drain the system, and a functional biomedical device in fluid communication with the first biological fluid container and the first biological fluid receiving container, the method comprising:

a) causing the precharge of gas to be transferred from the first biological fluid receiving container to the first biological fluid container without wetting the functional biomedical device; and
b) passing the fluid from the first biological fluid container, through the functional biomedical device, and collecting the filtered fluid.

35. The method defined in aspect 34, wherein the fluid is a liquid.

36. The method defined in aspect 35, wherein the liquid is a biological fluid.

37. The method defined in aspect 36, wherein the biological fluid is blood, and the functional biomedical device is a leukocyte reduction device.

38. A ventless, prechargeable, fluid processing system comprising:

a) a functional biomedical device having an inlet and an outlet and a porous medium interposed between said inlet and said outlet;

b) a first tubing connected at a second end in fluid communication with the inlet of the functional biomedical device and closed at its first end;

c) a second tubing connected at a first end in fluid communication with the outlet of the functional biomedical device;

d) a biological fluid receiving container connected in fluid communication with the second end of the second tubing; and

e) a precharge of gas in the second container sufficient to drain the system.

39. A method for processing a biological fluid using a processing system having a first biological fluid container containing a quantity of a biological fluid, a first biological fluid receiving container downstream of said first biological fluid container, and a functional biomedical device in fluid communication with the first biological fluid container and the first biological fluid receiving container, the method comprising:

a) introducing a precharge into the first biological fluid container; and

b) passing the biological fluid from the first biological fluid container, through the functional biomedical device, and collecting the leukocyte depleted biological fluid in the second container.

40. A method for processing a fluid using a processing system having a first biological fluid container, a first biological fluid receiving container downstream of said first biological fluid container, and a fluid filtration device in fluid communication with the first biological fluid container and the first biological fluid receiving container, the method comprising:

a) injecting a pre-determined amount of precharge into the first biological fluid container; and

b) passing the fluid from the first biological fluid container, through the fluid filtration device, and collecting the filtered fluid in the first biological fluid receiving container.

41. The method defined in aspect 40, wherein the fluid is a liquid.

42. The method defined in aspect 41, wherein the liquid is a biological fluid.

43. The method defined in aspect 42, wherein the biological fluid is blood or blood product, and the fluid filtration device is a leukocyte reduction device.

44. A method for processing a biological fluid using a processing system including at least one first biological fluid container, at least one first biological fluid receiving container downstream of the first biological fluid container precharged with a quantity of gas sufficient to drain the system, and at least one functional biomedical device in fluid communication with the at least one first biological fluid container and the at least one first biological fluid receiving container, the method comprising:

a) causing the precharge of gas to be transferred from the at least one biological fluid receiving container to the at least one first biological fluid container; and

b) passing the biological fluid from the at least one first biological fluid container, through the functional biomedical device, and collecting the filtered fluid in the at least one biological fluid receiving container.

45. A method for processing a biological fluid using a processing system having a first biological fluid container containing a fluid to be filtered, a first biological fluid receiving container downstream of said first biological fluid container, and a functional biomedical device in fluid communication with the first biological fluid container and the first biological fluid receiving container, the method comprising:

a) causing a precharge of gas sufficient to drain the system to be injected into the first biological fluid container; and

b) passing the biological fluid from the first biological fluid container, through the functional biomedical device and into the first biological fluid receiving container.

46. A method for processing a biological fluid using a processing system having a first biological fluid container, a diversion bag connected to the first biological fluid container and containing a precharge of gas sufficient to drain the system, a first biological fluid receiving container, and a functional biomedical device in fluid communication with the first biological fluid receiving container and the biological fluid container, the method comprising:

a) causing the precharge of gas to be transferred from the diversion bag to the first biological fluid container;

b) introducing blood into the first biological fluid container; and

c) passing the blood from the first biological fluid container, through the functional biomedical device.

47. A method for processing blood using a processing system having a first biological fluid container, a diversion bag connected to the first biological fluid container and containing a precharge of gas sufficient to drain the system, a first biological fluid receiving container, and a functional biomedical device in fluid communication with the first biological fluid receiving container and the first biological fluid container, the method comprising:

a) introducing blood into the first biological fluid container;
b) causing the precharge of gas to be transferred from the diversion bag to the first biological fluid container; and
c) passing the blood from the first biological fluid container, through the functional biomedical device.

**Claims**

1. A method for processing a biological fluid using a processing system having a first biological fluid container containing a quantity of biological fluid, a first biological fluid receiving container downstream of said first biological fluid container precharged with a quantity of gas sufficient to drain the system, and a functional biomedical device in fluid communication with the first biological fluid container and the first biological fluid receiving container, the method comprising:

a) causing the precharge of gas to be transferred from the first biological fluid receiving container to the first biological fluid container; and
b) passing the biological fluid from the first biological fluid container, through the functional biomedical device.

2. A method for processing a leukocyte containing biological fluid using a leukocyte reduction system having a first biological fluid container containing a quantity of a leukocyte containing biological fluid, a first biological fluid receiving container downstream of said first biological fluid container precharged with a quantity of gas sufficient to drain the system, and a leukocyte reduction device in fluid communication with the first biological fluid container and the first biological fluid receiving container, the method comprising:

a) causing the precharge of gas to be transferred from the first biological fluid receiving container to the first biological fluid container; and

b) passing the leukocyte containing biological fluid from the first biological fluid container, through the leukocyte reduction device.

3. A method for processing a biological fluid comprising:

a) passing a predetermined quantity of gas from a first biological fluid receiving container through a leukocyte reduction device into a first biological fluid container containing a leukocyte rich biological fluid; and
b) passing the leukocyte rich biological fluid from the first biological fluid container, through the leukocyte reduction device, into the first biological fluid receiving container.

4. A prechargeable fluid filtration apparatus comprising:

a) a functional biomedical device having an inlet and an outlet and a porous medium interposed between the inlet and the outlet;
b) a first tubing connected at one end in fluid communication with the inlet of the functional biomedical device;
c) a second tubing connected at one end in fluid communication with the outlet of the functional biomedical device;
d) a first biological fluid receiving container connected in fluid communication with the other end of the second tubing; and
e) a precharge of gas in the first biological fluid receiving container sufficient to drain the system, the precharge of gas is greater than the residual air found in the ventless biological filtration system in an empty collapsed state.

5. A ventless biological fluid processing system comprising:

a) a functional biomedical device having an inlet and an outlet and a porous medium interposed between said inlet and said outlet;
b) a first tubing having at least a first end and a second end, the second end of the first tubing connected to the inlet of the functional biomedical device;
c) a second tubing having at least a first end and a second end, and being connected at the first end in fluid communication with the outlet of the functional biomedical device;
d) a first biological fluid container connected in fluid communication with the first end of the first tubing;
e) a first biological fluid receiving container connected in fluid communication with the second end of the second tubing; and

f) a precharge of gas in the first biological fluid receiving container.

6. A method for processing a biological fluid using a processing system having a first biological fluid container containing a quantity of biological fluid, a first biological fluid receiving container downstream of said first biological fluid container precharged with a quantity of gas sufficient to drain the system, and a functional biomedical device in fluid communication with the first biological fluid container and the first biological fluid receiving container, the method comprising:

a) causing the precharge of gas to be transferred from the first biological fluid receiving container to the first biological fluid container by applying a vacuum about the first biological fluid container; and

b) passing the biological fluid from the first biological fluid container, through the functional biomedical device, and collecting the biological fluid in the first biological fluid receiving container.

7. A method for processing a fluid using a processing system having a first biological fluid container containing a quantity of biological fluid, a first biological fluid receiving container downstream of said first biological fluid container precharged with a quantity of gas sufficient to drain the system, and a functional biomedical device in fluid communication with the first biological fluid container and the first biological fluid receiving container, the method comprising:

a) causing the precharge of gas to be transferred from the first biological fluid receiving container to the first biological fluid container without wetting the functional biomedical device; and

b) passing the fluid from the first biological fluid container, through the functional biomedical device, and collecting the filtered fluid.

8. A ventless, prechargeable, fluid processing system comprising:

a) a functional biomedical device having an inlet and an outlet and a porous medium interposed between said inlet and said outlet;

b) a first tubing connected at a second end in fluid communication with the inlet of the functional biomedical device and closed at its first end;

c) a second tubing connected at a first end in fluid communication with the outlet of the functional biomedical device;

d) a biological fluid receiving container connected in fluid communication with the second end of the second tubing; and

e) a precharge of gas in the second container sufficient to drain the system.

9. A method for processing a biological fluid using a processing system having a first biological fluid container containing a quantity of a biological fluid, a first biological fluid receiving container downstream of said first biological fluid container, and a functional biomedical device in fluid communication with the first biological fluid container and the first biological fluid receiving container, the method comprising:

a) introducing a precharge into the first biological fluid container; and

b) passing the biological fluid from the first biological fluid container, through the functional biomedical device, and collecting the leukocyte depleted biological fluid in the second container.

10. A method for processing a fluid using a processing system having a first biological fluid container, a first biological fluid receiving container downstream of said first biological fluid container, and a fluid filtration device in fluid communication with the first biological fluid container and the first biological fluid receiving container, the method comprising:

a) injecting a pre-determined amount of precharge into the first biological fluid container; and

b) passing the fluid from the first biological fluid container, through the fluid filtration device, and collecting the filtered fluid in the first biological fluid receiving container.

11. A method for processing a biological fluid using a processing system including at least one first biological fluid container, at least one first biological fluid receiving container downstream of the first biological fluid container precharged with a quantity of gas sufficient to drain the system, and at least one functional biomedical device in fluid communication with the at least one first biological fluid container and the at least one first biological fluid receiving container, the method comprising:

a) causing the precharge of gas to be transferred from the at least one biological fluid receiving container to the at least one first biological fluid container; and

b) passing the biological fluid from the at least one first biological fluid container, through the functional biomedical device, and collecting the filtered fluid in the at least one biological fluid receiving container.

12. A method for processing a biological fluid using a processing system having a first biological fluid container containing a fluid to be filtered, a first biological fluid receiving container downstream of said first biological fluid container, and a functional biomedical device in fluid communication with the first biological fluid container and the first biological fluid receiving container, the method comprising:

> a) causing a precharge of gas sufficient to drain the system to be injected into the first biological fluid container; and
> b) passing the biological fluid from the first biological fluid container, through the functional biomedical device and into the first biological fluid receiving container.

13. A method for processing a biological fluid using a processing system having a first biological fluid container, a diversion bag connected to the first biological fluid container and containing a precharge of gas sufficient to drain the system, a first biological fluid receiving container, and a functional biomedical device in fluid communication with the first biological fluid receiving container and the biological fluid container, the method comprising:

> a) causing the precharge of gas to be transferred from the diversion bag to the first biological fluid container;
> b) introducing blood into the first biological flud container; and
> c) passing the blood from the first biological fluid container, through the functional biomedical device.

14. A method for processing blood using a processing system having a first biological fluid container, a diversion bag connected to the first biological fluid container and containing a precharge of gas sufficient to drain the system, a first biological fluid receiving container, and a functional biomedical device in fluid communication with the first biological fluid receiving container and the first biological fluid container, the method comprising:

> a) introducing blood into the first biological fluid container;
> b) causing the precharge of gas to be transferred from the diversion bag to the first biological fluid container; and
> c) passing the blood from the first biological fluid container, through the functional biomedical device.

**Fig-1A**

| Pre-Charge | |
| --- | --- |
| Blood | |
| Anticoagulant | |
| Preservative | |

**Fig-1**

**Fig-2**

**Fig-3**

Fig-4

Fig-5

Fig-6

Fig-7

_Fig-8_    _Fig-9_    _Fig-10_

Fig-11

Fig-12

Fig-13

*Fig-14A*

*Fig-14*    *Fig-15*    *Fig-16*

Fig-17

Fig-18

Fig-19

_Fig-20_     _Fig-20A_

_Fig-21_          _Fig-21A_          _Fig-21B_

_Fig-22_          _Fig-22A_          _Fig-22B_

Fig-23

Fig-23A

Fig-23B

EP 2 494 997 A2

Fig-24

30

_Fig-25_

*Fig-26*

**EP 2 494 997 A2**

## REFERENCES CITED IN THE DESCRIPTION

**Patent documents cited in the description**

- US 3892236 A, Djerassi
- US 5126054 A, Matkovich
- US 5451321 A, Matkovich
- US 5472621 A, Matkovich
- US 5527472 A, Bellotti
- US 5863436 A, Matkovich
- US 6802425 B2, Zia